# EUROPEAN PATENT SPECIFICATION

(11) **EP 3 250 183 B1**
(45) Date of publication and mention of the grant of the patent: **19.06.2024**
(21) Application number: 16701812.6
(22) Date of filing: 28.01.2016
(51) Int. Cl.: A61K 31/216, A61K 38/28, A61K 31/343, A61K 31/4168, A61K 31/428, A61K 31/465, A61K 31/485, A61K 31/57, A61M 31/00, A61K 9/00, A61P 13/10

(54) **VAGINAL DRUG DELIVERY DEVICE**
SYSTEM FÜR VAGINALE ARZNEIMITTELABGABE
SYSTÈME DE DISTRIBUTION DE MÉDICAMENT VAGINAL

(30) Priority: 30.01.2015 EP 15153322
(43) Date of publication of application: 06.12.2017
(60) Divisional application: 24171612.5
(73) Proprietor: LiGalli B.V., 2514 AC Den Haag (NL)
(72) Inventor: DE LAAT, Wilhelmus Nicolaas Gerardus Maria, 2514 AC Den Haag (NL); VAN DER VAART, Carl Huibert, 3707 BE Zeist (NL)
(74) Representative: van Someren, Petronella F. H. M.
(86) International application number: PCT/EP2016/051835
(87) International publication number: WO 2016/120402

(56) References cited:
- EP-A1- 1 278 494
- WO-A2-2004/096151
- WO-A2-2008/155589
- US-A1- 2006 084 848
- US-A1- 2013 066 302
- US-A1- 2013 220 337
- US-A1- 2014 296 834
- US-B2- 7 044 911

## Description

### FIELD OF THE INVENTION

The present invention relates to a vaginal drug delivery device for use in the treatment of medical conditions in women. The device enables "personalized" or "individualized medicine".

### BACKGROUND OF THE INVENTION

Vaginal ring systems are usually used for contraception and hormone replacement therapy. Different ring systems for these applications are known in the prior art.

### SUMMARY OF THE INVENTION

According to the invention it has now been found that other than hormonal drugs can also be administered and absorbed via the vagina. Administration via the vagina is in particular useful for compounds that have relatively low or negligible oral bio-availability, e.g. due to insufficient absorption, degradation in the gastro-intestinal (GI) tract (e.g. peptides and proteins), or other factors, for compounds with unwanted GI side-effects, for compounds with a narrow therapeutic range, for compounds with a short half-life (t½) that require frequent or high dosing, for compounds that require a complex temporal and/or dosing schedule for adequate efficacy, etc. Such compounds can be over-the-counter (OTC) compounds as well as prescription drugs.

The vaginal administration of oxybutynin is disclosed in WO01/70154, which relates to a trifluoropropylmethyl/dimethyl siloxane elastomer, which releases oxybutynin to the cervical region of a female to treat urinary incontinence. This ring leads to the continuous release of oxybutynin over a period of time. Since the oxybutynin diffuse out of the elastomer matrix the release cannot be interrupted or adapted. Continuous dosing of oxybutynin will lead to upregulation of the G-coupled receptor, thus decreasing the efficacy.

The invention thus relates to a vaginal drug delivery device configured for placement within the vagina, comprising a housing, at least one reservoir comprising an anticholinergic medicament, in particular oxybutynin, dispensing means and means for the controlled release of the anticholinergic compound from the reservoir into the vagina, wherein the device is a ring which comprises a first rigid member, a second rigid member, a first flexible member and a flexible part, wherein the first flexible member is made of an elastic material and comprises a recess to allow the first and second rigid members to move towards each other when the device is squeezed from the extended into the collapsed state, and wherein the controlled release is programmable according to a defined release pattern.

In another embodiment, the device of the invention further comprises means for gathering diagnostic data of a person carrying the device in her vagina.

In this embodiment, means for drug release and diagnosis are combined in the ring. Release of the compound from the ring is preferably controlled in response to diagnostic data of the user of the ring gathered by measuring means that are preferably located in, - or connected to, - the ring. For this, the device further comprises means for gathering data of a person carrying the device in her vagina. The means for the controlled release of the compound from the reservoir are then configured - either via handheld device / mobile device or independently/interactively by the ring itself -to act in response to physiological data of a person carrying the device in her vagina.

The means for the controlled release of the compound from the reservoir in the vaginal device of the invention can also be pre-programmed with a defined release pattern. The defined release pattern comprises for example pulsatile release, spiked release, timed release, intermittent release, or combinations thereof or any other desired release pattern. In a further embodiment, the release of the medicament can be "on demand". This means for example that amounts of medication can be added by the patient, based on her medical complaints or in response to results of her diagnostic sensor. This "on-demand" possibility can be combined with any of the aforementioned release patterns.

The means for gathering physiological data of a person carrying the device in her vagina may comprise but are not limited to means for measuring temperature, glucose levels in the transudate of the vaginal mucosa, physico-chemical properties of the cervical mucus (like viscosity, clarity quantity and moisture), means for monitoring hormone levels,-either in the cervical mucus or in the transudate of the vaginal mucosa-, in particular of the hormones FSH, LH, 17-β-estradiol and progesterone or combinations thereof, means for analyzing proteins or nucleic acids, such as DNA, means for detecting molecules, in particular signaling molecules, such as cytokines, means to monitor the (an)ovulatory cycle, means for measurement of hyaluronic acid, or combinations thereof.

In one embodiment, the invention relates to the vaginal device, which is loaded with the anticholinergic medicament, in particular oxybutynin. The medicament is then considered an integral part of the device.

Administration via a vaginal device according to the invention is in particular suitable for compounds that have relatively low or negligible oral bio-availability, unwanted side-effects in the gastrointestinal tract, a narrow therapeutic range and/or an efficacy that depends on a complex temporal administration schedule or a short half-life. Such compounds are anticholinergics (e.g. oxybutynin, solifenacin). Compounds that are released vaginally and are close to the target organ (like vagina, bladder, uterus and ovaries) will exert a "local" effect by simple diffusion and thus higher concentrations can be obtained, even with a lower dose. The administration approach of the present invention is thus particularly suitable for such compounds.

The present invention is in particular useful for oxybutynin. In addition to oxybutinin, its analogs, homologs or agonists thereof or related anticholinergic compounds having the same pharmaceutical effect can be administered via a vaginal ring.

### DETAILED DESCRIPTION

The invention thus relates to a new means of administration of certain anticholinergic medicament, in particular oxybutynin, via a device that is placed in the vagina and from which the compound is released in a controlled manner. The device comprises a housing in which the various components needed for the desired function are located. The housing can at the same time be the vehicle that is placed in the vagina. The device can have various functions but will in any case comprise a reservoir for anticholinergic medicament, in particular oxybutynin and means for dispensing the medicament into the vagina and means for doing so in a controlled manner. Additional functions comprise for example means that measure certain parameters in the vagina, such as temperature, glucose, medication levels, hormone levels, physico-chemical parameters of the cervical mucus etc.

The device can be a ring or any other form that can comfortably be inserted into and carried in the vagina. The device can thus have different forms and functions, next to serving as a vehicle that assures continued presence within the vagina. The device may for example be a ring that has a flexible part and a rigid part, preferably in the top portion of the ring. The device is configured in such a way that the thicker rigid part can be compressed and will be inserted first, the ring will then defold after insertion with the rigid part settling smoothly in the deepest part of the vagina (fornix posterior) and the outer parts of the ring in flexible contact with the vaginal wall to prevent expulsion and enhance absorption.

In one embodiment, the ring comprises a positioning member configured for placement within the vagina and a device comprising one or more components selected from drug reservoirs, diagnostic means and also means for dispensing the drug from a reservoir into the vagina, wherein according to the invention the drug reservoir is loaded with a anticholinergic medicament, in particular oxybutynin. Themedicament is preferably formulated such that it will be sufficiently stable during storage and the period that the ring is in place and can be formulated with an absorption enhancer, where necessary.

An important feature of the invention is that the release of the anticholinergic medicament, in particular oxybutynin, from the device can be controlled, even during presence of the device in the vagina, i.e. remotely. To achieve this the vaginal ring of the invention has electronic equipment to program any schedule of release of the medication. This allows for "personalized" or "individualized medicine". Schedules like cyclical, daily, nightly, hourly, or pulsed/ in spikes, on demand etc. can be programmed adapted to the user's needs.

The schedule can be pre-programmed and/or adjusted either remotely, based on wishes of the user or by the patient, a physician or nurse based on results of diagnostic tests transmitted to the outside world by the device, or by a direct adaptation in the release pattern by the device itself based on diagnostic measurements made by the device. The device is than suitably programmed such that a particular result of a measurement automatically leads to a corresponding adaptation of the release pattern of the compound. To achieve this, the device comprises measuring means, such as sensors, probes, etc. Suitable sensors are temperature sensors, pH sensors, glucose sensors or sensors that can measure physic-chemical properties of the cervical mucus ( viscosity, quantity, moisture, clarity) or biosensors for measuring biological compounds such as medication. In addition, information is stored in the device or can be sent to the device that instructs the device how to adapt the release in response to a measured parameter.

The invention is preferably used with the embodiment described in EP3153137A1. In another embodiment, the ring can have a ring shape, wherein the cross section of the ring is variable along the circumference of the ring. In one embodiment the ring, can be used in combination with the Intellicap^{™} technology as described in WO2011/039680. The Intellicap^{™} can be suitably used for harboring the release, control and diagnostic functions of the vaginal device.

The vaginal device of the invention contains a reservoir or a cartridge which can be filled with anticholinergic medicines that are absorbed vaginally at biologically active levels, either in the presence or the absence of an absorption enhancer or administered for vaginal topical drug delivery via programmable (either pre-programmed or by remote control) release.

The systemic drug delivery via the vagina has many general advantages as compared to oral administration, such as no nausea, no irritation of the gastrointestinal tract, elimination of hepatic first pass drug metabolism, prevention of enzymatic degradation of drugs in the gastrointestinal tract. As compared to parenteral administration the ring is less invasive. It is an excellent system for long term medication and has the advantages of high bioavailability enabling lower doses for drugs which are currently administered orally, high bioavailability, thus avoiding parenteral delivery as injection, infusion or subcutaneous delivery, and has the added advantage of self-empowerment by enabling self-administration, self-control of insertion and removal, leading to high convenience and high compliance. Obviously the user remains ambulatory and outpatient.

The present invention offers unique advantages in the treatment of bladder problems and/or situations that require more frequent and/or untimely and/or complex drug delivery schedules compared to separate single oral doses or separate single injections.

The present invention offers in particular excellent medication regulation for diseases that require stable drug serum levels for adequate disease control because the release from the device can be adapted to the need of the user at a particular moment.

The present invention also allows for pulsatile delivery of compounds.

The present invention also shows specific advantages for treatment of diseases that require a high compliance rate in drug intake. An inserted and activated the ring will have a 100% compliance rate during the stay in the vagina, which can be 1-4 weeks.

The present invention is perfectly suited for drug delivery at untimely hours (such as nightly), for pulsed drug delivery, such as the treatment of OAB.

Various drug delivery regimes can also be combined. The device may e.g. contain multiple reservoirs for different medicaments. Another example of combined delivery is pulsed drug delivery on demand or preprogrammed, on top of a constant or steadily declining baseline level, e.g. OAB.

The present invention also allows for drug delivery close to the anatomically adjacent organs like the uterus, the vagina and the bladder. One example is a vaginal device, such as a ring, with medication for OAB.

The device of the invention is in particular used for treating overactive bladder (OAB) or urge incontinence with oxybutynin or another anticholinergic compound. Oral anticholinergics have a short t½, and a strong first-pass effect. They must be frequently and/or highly dosed and oxybutynin's primary metabolite, N-desethyloxybutynin (DEO), causes strong side-effects, particularly dry mouth, after oral administration. When oxybutynin (or another similar compound) is administered by means of a vaginal drug delivery device according to the invention, the therapeutic window of oxybutynin is widened by improving the efficacy/side-effect ratio, by lowering the side-effects via avoiding the first-pass effect and thus lowering the metabolite level, by improving the efficacy by allowing use of a higher dose, by benefiting from the "local" diffusion effect close to the bladder and by benefiting from the possibility to program the device for pulsatile delivery of the anticholinergic, in particular oxybutynin. Continuous dosing of antagonists will lead to upregulation of the G-coupled receptor, thus decreasing the efficacy. Upregulation will be avoided by pulsatile administration.

According to the invention oxybutynin is used for the treatment of urinary and bladder problems, in particular urge incontinence, wherein oxybutynin is administered to a person in need of treatment via the vagina, in particular by means of a vaginal device, more in particular the vaginal device as disclosed herein. When oxybutynin is used for the preparation of a medicament for the treatment of urinary and bladder problems, in particular urge incontinence, the medicament is formulated as a vaginal device comprising oxybutynin or a related compound. A method for the treatment of urinary and bladder problems, in particular urge incontinence, comprises administration of oxybutynin or a related compound to a person in need of treatment via the vagina, in particular by means of a vaginal device, more in particular the vaginal device as disclosed herein. In particular, the oxybutynin or related compound is released from the device in a pulsatile release pattern.

The oxybutynin, or any other anticholinergic, like solfenacin, is suitably in the form of a liquid which is able to dispense the drug from the vaginal drug delivery system to the vaginal cavity. For this the active ingredient (oxybutynin) should be locally in solution or must be capable of being solved when administered as a suspension. In order to facilitate release from the device by penetrating the membrane adjustments to the pH can be made or a solubilizing agent, such as a cyclodextrin derivative, can be added. In one embodiment the oxybutynin containing liquid contains oxybutynin base, hydrochloric acid salt, or any other biocompatible salt, crystal, co-crystal or complex or mixtures thereof, such as sulphate, phosphate, complexes with cyclodextrin, for example hydroxypropyl-beta-cyclodextrin.

The oxybutynin containing liquid can be a true solution, a colloidal solution, an emulsion, a micro-emulsion, a nano-emulsion, a suspension, a micro-suspension or a nano-suspension. The oxybutynin containing liquid can be a Newtonian or non-Newtonian liquid of low to medium/high viscosity. The characteristics of the device determine the maximum viscosity that can still be pumped from the vaginal drug delivery system.

The oxybutynin containing liquid suitably comprises aqueous or non-aqueous biocompatible solvents or mixtures thereof. It suitably comprises a high concentration of active substance allowing optimal drug absorption from the vaginal cavity, such as higher than 80 mg/ml.

The oxybutynin containing liquid is formulated such that no drug precipitation occurs in the vaginal environment. This can for example be prevented by adjusting the pH and/or adding a small amount of polyethylene glycol of propylene glycol.

The oxybutynin containing liquid is preferably stable for at least 12 months under the drug product storage condition and at least stable for 1 month at body temperature.

In the context of oxybutynin the device can include the function of measuring electromyography (EMG) signals of the bladder. These signals can predict early bladder contractions and the device can subsequently release oxybutynin "on demand".

In addition to the above, the device can be used for topical drug delivery. Topical drug delivery via a vaginal device has many general advantages, in particular reduced side effects from reducing or eliminating systemic exposure and reduced dose by delivery to site of action.

The device that is used according to the invention can also be used for diagnostic purposes. The diagnostic data may be used to adapt the release of the compound or compounds from the device.

The ring is in contact with the vaginal mucosa and the measurements with the sensors are either made in the transudate of the vaginal wall and/or the cervical mucus.

Certain parameters measured in the vagina can be used to calculate the level of the parameter in serum. For this a conversion has to be made. The skilled person knows how to do this.

Another function that can be included in the device are measurements of the physico-chemical properties of the cervical mucus (like quantity, moisture, clarity and viscosity).

In order to determine whether a woman is compliant in wearing the device a temperature sensor can be included. If the device is located in the vagina the temperature will record about 38°C and these results can be recorded and transmitted, either to an outside computer or handheld device. In addition, the device can also keep a log of the amount of compound that is released, if applicable.

According to the present invention, the device is a ring, in particular a ring which contains a flexible part and a rigid part. In this embodiment, the flexible part of the ring has several proposed shapes and may also contain an antenna for communication with the ring, e.g. related to programming, or for transmission of data, generated by the diagnostic systems in the ring to the outside and/or for receiving commands from outside the body. The electronics are preferably based on the Intellicap^{™} technology as described in WO2011/039680. Furthermore, the ring contains a special pump and container/cartridge and communication and data transfer means.

The ring of the invention is designed in such a way that it is safe, causes no lesion to the vaginal epithelium and cervix at insertion, removal or use of the device, is produced from material(s) of approved medical grade, is non-irritating, non-allergenic, has no risk of breakage, no risk of disintegration of electronic parts into the vagina, and comprises a built-in prevention of erroneous dose delivery.

The outer surface of the ring is suitably smooth to avoid irritation of the vaginal wall. The inner surface of the ring is preferably smooth to allow convenient intercourse. The elastic property of the ring offers a stabile positioning of the ring in the vagina. Suitably, the electronic parts with actuator are connected to the medication reservoir and housed together within the ring without compromising the round or oval structure of the ring. The configuration of the ring is such that the correct insertion is unequivocal.

The ring that is used in the invention can be pre-programmed for personalized medication or programmed and program-adjusted from a personal external transmitter device using Near Field Communication (NFC) technology, Bluetooth or other systems. In addition, the ring can be configured such that data gathered by the diagnostic means in the ring is directly translated into an adaptation of the release parameters.

The ring can collect data on the administered medication for evaluation by the physician. Also diagnostic data can be transmitted and stored when sensors are used in the ring. Electronics and mechanics suitably form a rigid and thicker portion of the ring that will smoothly settle in the deeper part of the vagina (fornix posterior) after insertion.

The diagnostic results can be communicated with an outside transmitter device like computer, smart phone or network, or used in autonomous feed-back mode to trigger release from the medication reservoir.

The possibility of a "lab-on-a-chip", which includes drug assays to establish the optimal serum levels of medications mentioned herein, will allow remotely controlled adjustment of the medication or the interaction of diagnostic and drug delivery parts of the ring.

In a preferred embodiment all functionalities of the drug delivery part and diagnostic part are therefore combined to provide an independently and interactive system that can adjust the drug delivery dose based on the results of the diagnostics.

For drug delivery, the ring comprises one or more components selected from a drug storage space (reservoir), means for regulated drug release, a transport system to the drug release site, a membrane or micro-pore surface at the drug delivery site, means for variable, continuous, interrupted or single drug delivery, means for drug delivery in fluid solution, in (adhesive) gel composition, in a foam composition, in a fluid composition to restore or protect vaginal ecology (e.g. pH) etc.

The functional components can suitably be integrated in an electronic capsule. Such electronic capsule can for example comprise one or more of the following elements: an internal connection electronic system to diagnostic sensors, an internal connection to drug release parts (storage, transport, contact surface), means for analysis of diagnostic parameters, means for storage of diagnostic data, means for data transmission to external data equipment, a receiver for external control of function, preferably embedded in a protected housing, alert signaling at malfunction, automatically switch off at malfunction etc.

For optimizing the conditions for use, the ring is configured such that it is in place intra-vaginally for 1-4 weeks, that intercourse remains possible at use, that no lesions of the partner occur at intercourse, that there is no adverse effect of the drug delivered by the device on the partner, that there is no expulsion of the device and no induced or involuntary discharge of the active compound, that the position of the device in the vagina allows correct monitoring, etc.

The ring is especially accepted by the female because of self-empowerment of use, by clear instructions, by self-insertion and self removal, by comfort of use (no pain or irritation) during 1-4 weeks, by the partner's acceptance and non-interference during coitus, and by clear (pre-programmed) use or easy adjustment by tele-command.

According to the invention a vaginal ring comprises a positioning member configured for placement within the vagina and an electronic device comprising one or more components selected from drug reservoirs, diagnostic systems and systems for dispensing the drug from a reservoir into the vagina, wherein the drug reservoir is loaded with a medicament and the medicament is preferably formulated such that it will be sufficiently stable during storage and the period that the ring is in place and is optionally formulated with an absorption enhancer. The compound has relatively low or negligible oral bio-availability, e.g. due to insufficient absorption, degradation in the gastro-intestinal (GI) tract (e.g. peptides and proteins), or other factors, or unwanted GI side-effects, a narrow therapeutic range, requires for efficacy a complex temporal administration schedule.

Administration of biologically active compounds via a vaginal device according to the invention allows for any administration schedule. In contrast to the known vaginal rings used for the administration of hormones, in which the compound is contained in a polymeric matrix and released therefrom in a manner dictated by the location and the concentration of the compound and the polymer, the presence of a reservoir in the ring device allows administration of any dosage. Moreover, even during the presence of the device in the vagina the dosage and release pattern can be adapted, for example in response to measurements made in the vagina made by diagnostic means in the device.

In the present application the words "device" and "ring" are used interchangeably.

The present invention will now be further illustrated in the Examples that follow. The Examples are given for illustration purposes only and are not intended to limit the invention in any way.

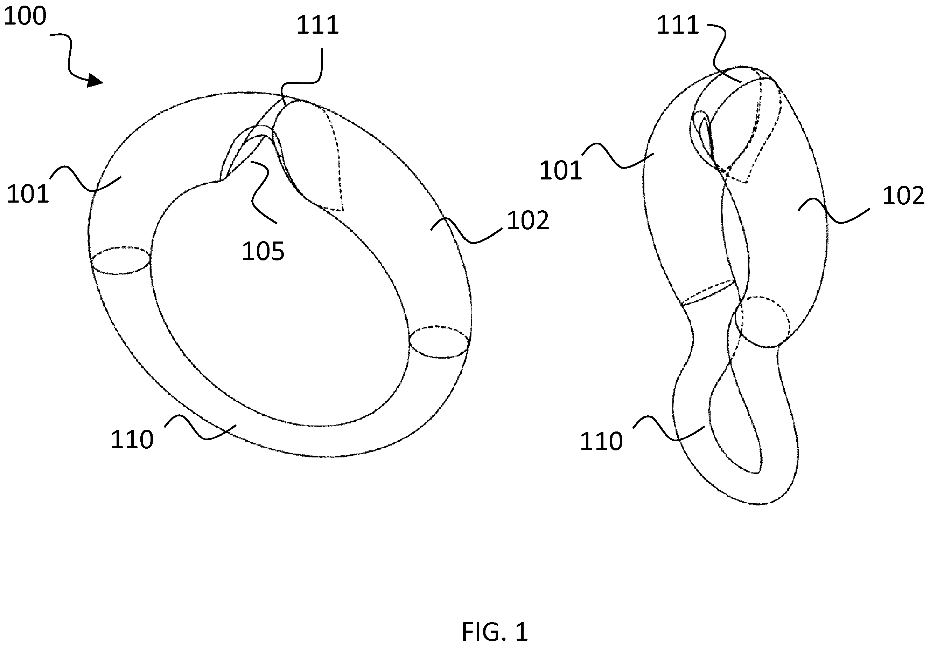

Figure 1 illustrates the shape of the device used in accordance with the present invention in an extended (left) and collapsed state (right). This shape comprises a first rigid member 101, a second rigid member 102, a first flexible member 111, and a flexible part 110. Here, first flexible member 111 is made of an elastic material and comprises a recess 105 to allow first and second rigid member 101, 102 to move towards each other when device 100 is squeezed into the collapsed state.

The functional parts of the drug delivery and/or diagnostic mechanism are incorporated into the rigid members 101, 102, whereas the (electrical) interconnect can be accommodated in the flexible member 111 or flexible part 110.

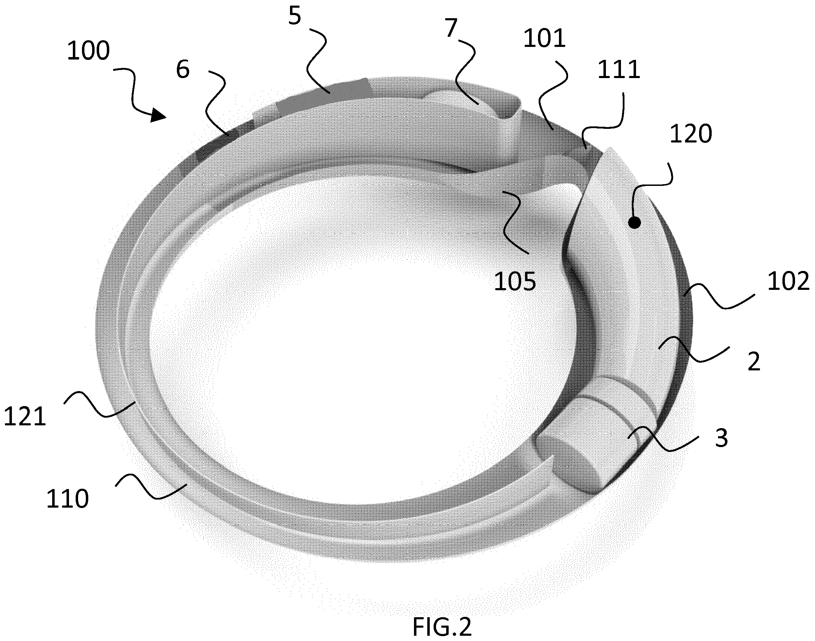

Figure 2 illustrates the device of figure 1 showing an exemplary arrangement of some of the components of the device. Here, it can be seen that the functional parts of the drug delivery mechanism, i.e. battery 7, controller 6, pump 3, reservoir 2, and sensor 5 are incorporated in first rigid member 101 and second rigid member 102. Moreover, an opening 120 can be identified through which the medicament held in reservoir 2 is pumped out by pump 3. This opening will be located at such a place on the outer perimeter of the ring to guarantee optimal contact with the vaginal mucosa. Furthermore, an electrical interconnect 121 can be seen that connects the various components to each other and to battery 7.

In figure 2, a transceiver may be incorporated in controller 6 or may alternatively be arranged as a separate component in first rigid member 101. This transceiver allows data communication between controller 6 and external systems or devices for remote control or for exchanging data such as measurement data.

First rigid member 101 is provided with an opening or membrane to enable sensor 5 to perform a predefined measurement. Similar openings or membranes can be provided if additional sensors are used which may or may not be arranged in first rigid member 101. These openings will be located at such places on the outer or inner perimeter of the ring to guarantee optimal contact with the vaginal mucosa or cervical mucus.

## Claims

1. A vaginal drug delivery device configured for placement within the vagina, comprising a housing, at least one reservoir comprising an anticholinergic medicament, in particular oxybutynin, dispensing means and means for the controlled release of the anticholinergic compound from the reservoir into the vagina,
wherein the device is a ring which comprises a first rigid member (101), a second rigid member (102), a first flexible member (111) and a flexible part (110), wherein the first flexible member is made of an elastic material and comprises a recess (105) to allow the first and second rigid members (101, 102) to move towards each other when the device (100) is squeezed from the extended into the collapsed state, and
wherein the controlled release is programmable according to a defined release pattern.

2. Vaginal device as claimed in claim 1, wherein the defined release pattern is pre-programmed.

3. Vaginal device as claimed in claim 1 or 2, wherein the defined release pattern is selected from pulsatile release, spiked release, timed release, intermittent release, or combinations thereof.

4. Vaginal device as claimed in any one of the claims 1 to 3, wherein the release is according to a pre-programmed release schedule which is selected from cyclical, daily, nightly, hourly.

5. Vaginal device as claimed in any one of the claims 1 to 4, wherein the defined release pattern is on top of a constant or steadily declining baseline level.

6. Vaginal device as claimed in any one of the claims 1 to 5, wherein the release is adjustable to the user's needs.

7. Vaginal device as claimed in any one of the claims 1 to 6, wherein the means for the controlled release of the compound from the reservoir are operated remotely.

8. Vaginal device as claimed in claim 7, wherein the means for the controlled release of the compound from the reservoir are operated remotely based on the wishes of the user.

9. Vaginal device as claimed in any one of the claims 1 to 8, wherein the release is on demand.

10. A vaginal drug delivery device as claimed in any one of the claims 1 to 9, further comprising means for gathering physiological data of a person carrying the device in her vagina.

11. Vaginal device as claimed in claim 10, wherein the anticholinergic medicament is oxybutynin and the means for gathering diagnostic data of a person carrying the device in her vagina comprise means for measuring electromyography (EMG) signals of the bladder.

12. Vaginal device as claimed in claim 11, wherein the means for the controlled release of the compound from the reservoir are operated remotely based on results of diagnostic tests transmitted to the outside world by the device.

13. Vaginal device as claimed in claim 12, wherein the means for the controlled release of the compound from the reservoir are operated by the person carrying the device in her vagina, a nurse or a physician.

14. Vaginal device as claimed in any one of the claims 1 to 13, wherein the anticholinergic compound, in particular oxybutynin, is for use in the treatment of urge incontinence or overactive bladder (OAB).

15. Vaginal device as claimed in any one of the claims 1 to 14, wherein the adjustment of the release is done from a personal external transmitter device, in particular using Near Field Communication technology or Bluetooth.

## Patentansprüche

1. Vaginale Arzneimittelabgabevorrichtung, die für eine Platzierung innerhalb der Vagina konfiguriert ist, umfassend ein Gehäuse, mindestens ein Reservoir, umfassend ein anticholinerges Medikament, insbesondere Oxbutynin, Ausgabemittel und Mittel für die kontrollierte Freigabe der anticholinergen Verbindung aus dem Reservoir in die Vagina,
wobei die Vorrichtung ein Ring ist, der ein erstes starres Element (101), ein zweites starres Element (102), ein erstes flexibles Element (111) und einen flexiblen Teil (110) umfasst, wobei das erste flexible Element aus einem elastischen Material hergestellt ist und eine Aussparung (105) umfasst, um zu ermöglichen, dass sich das erste und das zweite starre Element (101, 102) aufeinander zu bewegen, wenn die Vorrichtung (100) von dem ausgefahrenen in den zusammengeklappten Zustand zusammengedrückt wird, und
wobei die gesteuerte Freigabe gemäß einem definierten Freigabemuster programmierbar ist.

2. Vaginale Vorrichtung nach Anspruch 1, wobei das definierte Freigabemuster vorprogrammiert ist.

3. Vaginale Vorrichtung nach Anspruch 1 oder 2, wobei das definierte Freigabemuster aus einer pulsierenden Freigabe, einer versetzten Freigabe, einer zeitgesteuerten Freigabe, einer intermittierenden Freigabe oder Kombinationen davon ausgewählt ist.

4. Vaginale Vorrichtung nach einem der Ansprüche 1 bis 3, wobei die Freigabe gemäß einem vorprogrammierten Freigabezeitplan erfolgt, der aus zyklisch, täglich, nächtlich, stündlich ausgewählt ist.

5. Vaginale Vorrichtung nach einem der Ansprüche 1 bis 4, wobei das definierte Freigabemuster auf einem konstanten oder stetig abnehmenden Grundlinienniveau liegt.

6. Vaginale Vorrichtung nach einem der Ansprüche 1 bis 5, wobei die Freigabe auf die Bedürfnisse des Benutzers einstellbar ist.

7. Vaginale Vorrichtung nach einem der Ansprüche 1 bis 6, wobei die Mittel für die kontrollierte Freigabe der Verbindung aus dem Reservoir ferngesteuert werden.

8. Vaginale Vorrichtung nach Anspruch 7, wobei die Mittel für die kontrollierte Freigabe der Verbindung aus dem Reservoir basierend auf den Wünschen des Benutzers ferngesteuert werden.

9. Vaginale Vorrichtung nach einem der Ansprüche 1 bis 8, wobei die Freigabe auf Anforderung erfolgt.

10. Vaginale Arzneimittelabgabevorrichtung nach einem der Ansprüche 1 bis 9, ferner umfassend Mittel zum Sammeln physiologischer Daten einer Person, die die Vorrichtung in ihrer Vagina trägt.

11. Vaginale Vorrichtung nach Anspruch 10, wobei das anticholinerge Medikament Oxybutynin ist und die Mittel zum Sammeln diagnostischer Daten einer Person, die die Vorrichtung in ihrer Vagina trägt, Mittel zum Messen von Signalen einer Elektromyografie (EMG) der Blase umfassen.

12. Vaginale Vorrichtung nach Anspruch 11, wobei die Mittel für die kontrollierte Freigabe der Verbindung aus dem Reservoir basierend auf Ergebnissen von diagnostischen Tests, die durch die Vorrichtung an die Außenwelt übertragen werden, ferngesteuert werden.

13. Vaginale Vorrichtung nach Anspruch 12, wobei die Mittel für die kontrollierte Freigabe der Verbindung aus dem Reservoir durch die Person, die die Vorrichtung in ihrer Vagina trägt, einen Pfleger oder einen Arzt gesteuert werden.

14. Vaginale Vorrichtung nach einem der Ansprüche 1 bis 13, wobei die anticholinerge Verbindung, insbesondere Oxybutynin, zur Verwendung in der Behandlung von Dranginkontinenz oder einer überaktiven Blase (OAB) dient.

15. Vaginale Vorrichtung nach einem der Ansprüche 1 bis 14, wobei die Einstellung der Freigabe von einer persönlichen externen Sendervorrichtung, insbesondere unter Verwendung der Nahfeldkommunikationstechnologie oder Bluetooth, erfolgt.

## Revendications

1. Dispositif vaginal d'administration de médicament conçu pour être placé à l'intérieur du vagin, comprenant un logement, au moins un réservoir comprenant un médicament anticholinergique, en particulier l'oxybutynine, un moyen de distribution et un moyen de libération commandée du composé anticholinergique du réservoir dans le vagin,
dans lequel le dispositif est un anneau qui comprend un premier élément rigide (101), un second élément rigide (102), un premier élément flexible (111) et une partie flexible (110), dans lequel le premier élément flexible est constitué d'un matériau élastique et comprend un évidement (105) pour permettre aux premier et second éléments rigides (101, 102) de se déplacer l'un vers l'autre lorsque le dispositif (100) est pressé de l'état déployé à l'état replié, et
dans lequel la libération commandée est programmable selon un mode de libération défini.

2. Dispositif vaginal selon la revendication 1, dans lequel le mode de libération défini est préprogrammé.

3. Dispositif vaginal selon la revendication 1 ou 2, dans lequel le mode de libération défini est choisi parmi la libération pulsatile, la libération par pics, la libération temporisée, la libération intermittente ou leurs combinaisons.

4. Dispositif vaginal selon l'une quelconque des revendications 1 à 3, dans lequel la libération s'effectue selon un programme de libération préprogrammé qui est choisi parmi les éléments suivants : cyclique, quotidien, nocturne, horaire.

5. Dispositif vaginal selon l'une quelconque des revendications 1 à 4, dans lequel le mode de libération défini se situe au-dessus d'un niveau de base constant ou progressivement décroissant.

6. Dispositif vaginal selon l'une quelconque des revendications 1 à 5, dans lequel la libération est réglable selon les besoins de l'utilisateur.

7. Dispositif vaginal selon l'une quelconque des revendications 1 à 6, dans lequel le moyen de libération commandée du composé à partir du réservoir est actionné à distance.

8. Dispositif vaginal selon la revendication 7, dans lequel le moyen de libération commandée du composé à partir du réservoir est actionné à distance en fonction des souhaits de l'utilisateur.

9. Dispositif vaginal selon l'une quelconque des revendications 1 à 8, dans lequel la libération se fait à la demande.

10. Dispositif vaginal d'administration de médicament selon l'une quelconque des revendications 1 à 9, comprenant en outre un moyen de collecte des données physiologiques d'une personne portant le dispositif dans son vagin.

11. Dispositif vaginal selon la revendication 10, dans lequel le médicament anticholinergique est l'oxybutynine et le moyen de collecte de données de diagnostic d'une personne portant le dispositif dans son vagin comprend un moyen de mesure de signaux d'électromyographie (EMG) de la vessie.

12. Dispositif vaginal selon la revendication 11, dans lequel le moyen de libération commandée du composé à partir du réservoir est actionné à distance en fonction de résultats de tests de diagnostic transmis au monde extérieur par le dispositif.

13. Dispositif vaginal selon la revendication 12, dans lequel le moyen de libération commandée du composé à partir du réservoir est actionné par la personne portant le dispositif dans son vagin, par une infirmière ou par un médecin.

14. Dispositif vaginal selon l'une quelconque des revendications 1 à 13, dans lequel le composé anticholinergique, en particulier l'oxybutynine, est destiné à être utilisé dans le traitement de l'incontinence par impériosité ou de la vessie hyperactive (OAB).

15. Dispositif vaginal selon l'une quelconque des revendications 1 à 14, dans lequel le réglage de la libération est effectué à partir d'un dispositif émetteur externe personnel, en particulier à l'aide d'une technologie de communication en champ proche ou Bluetooth.
